# EUROPEAN PATENT SPECIFICATION

(11) **EP 2 537 832 B1**
(45) Date of publication and mention of the grant of the patent: **12.08.2015**
(21) Application number: 10857477.3
(22) Date of filing: 28.12.2010
(51) Int. Cl.: C07D 239/34

(54) **METHOD FOR PREPARING (E)-METHYL 2-[2-(6-CHLOROPYRIMIDIN-4-YLOXY)PHENYL]-3-METHOXYACRYLATE**
VERFAHREN ZUR HERSTELLUNG VON (E)-METHYL 2-[2-(6-CHLOROPYRIMIDIN-4-YLOXY)PHENYL]-3-METHOXYACRYLAT
MÉTHODE DE PRÉPARATION DE 2-[2-(6-CHLOROPYRIMIDIN-4-YLOXY)PHÉNYL]-3-MÉTHOXYACRYLATE DE (E)-MÉTHYLE

(30) Priority: 26.09.2010 CN 201010295293
(43) Date of publication of application: 26.12.2012
(73) Proprietor: Chongqing Unisplendour Chemical Co., Ltd., Chongqing 402160 (CN)
(72) Inventor: YAO, Rujie, Chongqing 402160 (CN); JIN, Haiqin, Chongqing 402160 (CN); ZHOU, Chuilong, Chongqing 402160 (CN); LIU, Dan, Chongqing 402160 (CN); YANG, Jianchuan, Chongqing 402160 (CN); HU, Jie, Chongqing 402160 (CN)
(74) Representative: Witte, Weller & Partner Patentanwälte mbB
(86) International application number: PCT/CN2010/080373
(87) International publication number: WO 2012/037764

(56) References cited:
- WO-A1-92/08703
- WO-A1-98/07707
- CN-A- 1 062 139
- CN-A- 1 228 086
- CHUAN, YONGMING ET AL.: 'Process Improvement on the Synthesis of (E)-2-[2-(6-chloropyrimidin-4-yloxy)phenyl] -3-methoxyacrylate' CHINESE JOURNAL OF SYNTHETIC CHEMISTRY vol. 15, no. 6, 2007, pages 798 - 800, XP008169075

## Description

This application claims the priority of China Patent Application No. 201010295293.2, filed with the Patent Office of China on September 26, 2010, titled "Method for preparing (E)-methyl 2-[2-(6-chloropyrimidin-4-yloxy) phenyl]-3-methoxyacrylate.

### Field of the invention

The present invention relates to the field of germicide, especially to a method for preparing (E)-methyl 2-[2-(6-chloropyrimidin-4-yloxy)phenyl]-3-methoxyacrylate.

### Background of the invention

Azoxystrobin is a germicide of the methyl methacrylate family, which has good activities against most fungal diseases, such as powdery mildew, rust disease, glume blight, net blotch, downy mildew, rice blast, and the like. Azoxystrobin is widely used in spraying stems, leaves, and treating seeds, as well as treating the soil. When used under recommended doses, azoxystrobin is safe to crops without phytotoxicity, and will not cause ground water pollution, thus is an environmentally friendly germicide.

(E)-Methyl 2-[2-(6-chloropyrimidin-4-yloxy)phenyl]-3-methoxyacrylate is an important intermediate for the synthesis of azoxystrobin, the structure of which is shown in formula (I). The method for preparing (E)-methyl 2-[2-(6-chloropyrimidin-4-yloxy)phenyl]-3-methoxyacrylate is generally as the following: first synthesizing methyl 2-[2-(6-chloropyrimidin-4-yloxy)phenyl]-3,3-dimethoxypropionate, the structure of which is shown in formula (II) by using methyl 3,3-dimethoxy-2-(2-hydroxyphenyl)propionate and 4,6-dichloropyrimidine as starting materials, then dealcoholizing one molecule of methanol from the methyl 2-[2-(6-chloropyrimidin-4-yloxy)phenyl]-3,3-dimethoxypropionate.

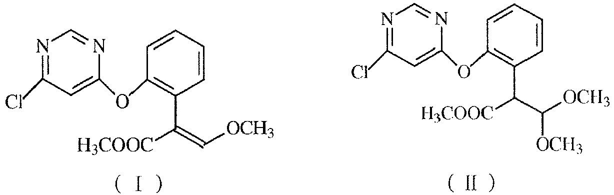

China patent application No. 97118615.4 discloses a method for preparing benzofuranone, in which a method of preparing (E)-methyl 2-[2-(6-chloropyrimidin-4-yloxy)phenyl]-3-methoxyacrylate using anhydrous potassium sulfate as a catalyst is disclosed, in such a method, the catalyst is applied directly in the reaction, the catalytic efficiency is low, and the yield of the reaction is low.

In the master's thesis "the synthesis of Azoxystrobin and the process optimization" by MIU Chengping of Zhejiang University (Zhejiang University, 2004.), a method for preparing (E)-methyl 2-[2-(6-chloropyrimidin-4-yloxy) phenyl]-3-methoxyacrylate is disclosed, which is specifically consisted of: dissolving methyl 3,3-dimethoxy-2-(2-hydroxyphenyl)propionate with DMF, adding 4,6-dichloropyrimidine and a base, then performing the first reaction by heating to prepare methyl 2-[2-(6-chloropyrimidin-4-yloxy)phenyl]-3,3-dimethoxypropionate; purifying the product from the first reaction, then performing the second reaction by adding a catalyst into the product and heating under vacuum, the mixture obtained in the second reaction is dissolved with dichloromethane, filtered, washed by water, dried and desolventized, to obtain (E)-methyl 2-[2-(6-chloropyrimidin-4-loxy)phonyl]-3-methoxyacrylate, in the above methods, the base is added directly into the reaction system as a catalyst, the problems of low catalytic efficiency and low reaction yield still exist, thereby the yield of (E)-methyl 2-[2-(6-chloropyrimidin -4-yloxy)phenyl]-3-methoxyacrylate is effected.

Chuan, Yongming et al.: "Process Improvement of the Synthesis of (E)-methyl 2-[2-(6-chloropyrimidin-4-yloxy)phenyl]-3-methoxyacrylate", Chinese Journal of Synthetic Chemistry, Vol. 15, No. 6, 2007, pp. 798-800 discloses preparation of said compound with 3,3-dimethoxy-2-(2-hydroxphenyl)proprionate and methyl 2-[2-(6-chloropyrimidin-4-yloxy)phenyl]-3,3-dimethoxy-proprionate as intermediate products.

### Summary of the invention

The technical problem to be solved by the present invention is to provide a method for preparing (E)-methyl 2-[2-(6-chloropyrimidin-4-yloxy)phenyl]-3-methoxyacrylate. The yield can be improved by using this method.

For this purpose, the present invention provides a method for preparing (E)-methyl 2-[2-(6-chloropyrimidin-4-yloxy)phenyl]-3-methoxyacrylate, which comprises
a. calcining, grinding and heating a basic catalyst to activate it, obtaining an activated catalyst;
b. performing a substitution reaction between methyl 3,3-dimethoxy-2-(2-hydroxyphenyl)propionate and 4,6-dichloropyrimidine in the presence of said activated catalyst, to obtain methyl 2-[2-(6-chloropyrimidin-4-yloxy)phenyl]-3,3- dimethoxypropionate; and
c. dealcoholizing the obtained methyl 2-[2-(6-chloropyrimidin-4-yloxy)phenyl]-3,3-dimethoxypropionate, to obtain (E)-methyl 2-[2-(6-chloropyrimidin-4-yloxy)phenyl]-3-methoxyacrylate.

The temperature of the calcining is 300°C~450°C.

The duration of the calcining is 2.5h~4h.

The activated catalyst is selected from the group consisting of K₂CO₃, Na₂CO₃, NaHCO₃, KHSO₄ and KHCO₃.

Preferably, the temperature of the heating activation is 70°C~130°C, and the pressure thereof is 20mmHg~50mmHg.

Preferably, the particle size of the activated catalyst is 50 mesh~120 mesh.

Preferably, the molar ratio of methyl 3,3-dimethoxy-2-(2-hydroxyphenyl)propionate, 4,6-dichloropyrimidine to the activated catalyst in step b is 1:1.2~2.2:1.2~2.2.

Preferably, the method further comprises a step of purifying (E)-methyl 2-[2-(6-chloropyrimidin-4-yloxy) phenyl]-3-methoxyacrylate, said purifying consists of:

performing a decolorizing treatment to (E)-methyl 2-[2-(6-chloropyrimidin-4-yloxy) phenyl]-3-methoxyacrylate;

dissolving the decolorized (E)-methyl 2-[2-(6-chloropyrimidin-4-yloxy)phenyl]-3 -methoxyacrylate using a mixed solution of an ester compound and an alkane compound, and cooling the same for crystallization, wherein the ester compound is isopropyl acetate or ethyl acetate, the alkane compound is cyclohexane or n-hexane, the weight ratio of the ester compound to the alkane compound in the mixed solution is 1.4~2.8:1, and the weight ratio of the decolorized (E)-methyl 2-[2-(6-chloropyrimidin-4-yloxy)phenyl]-3-methoxyacrylate to the mixed solution is 1:0.5~1.2.

Preferably, the decolorizing treatment is specifically consisted of the following steps: dissolving (E)-methyl 2-[2-(6-chloropyrimidin-4-yloxy)phenyl]-3-methoxyacrylate using isopropyl acetate or ethyl acetate, then adding activated charcoal.

Preferably, the weight ratio of (E)-methyl 2-[2-(6-chloropyrimidin-4-yloxy)phenyl]-3-methoxyacrylate to the activated charcoal is 1:0.05~0.25.

The present invention provides a method for preparing (E)-methyl 2-[2-(6-chloropyrimidin-4-yloxy)phenyl]-3-methoxyacrylate, in comparison with existing preparation methods, for the present invention, the catalyst is subjected to activating treatment before preparing methyl 2-[2-(6-chloropyrimidin-4-yloxy)phenyl]-3,3-dimethoxypropionate, said activating treatment comprises calcining the base catalyst first to remove the crystal water in the catalyst and avoid the participation of water in the substitution reaction, while the catalyst forms loose porous structure during the calcining, contributing to increase the contact area between the catalyst and the staring materials; performing grinding and heating activation orderly upon the basic catalyst, in order to increase the activation energy of the catalyst and to increase the contact area between the catalyst and the reaction materials in the solid-liquid two-phase reaction system. Therefore, after treating the basic catalyst in advance according to the method of the present invention, the catalytic efficiency and the conversion rate of the starting materials within the same reaction time were improved due to the increase of the catalytic activation energy, thus the product yield of the substitution reaction is increased, thereby the yield of the (E)-methyl 2-[2-(6-chloropyrimidin-4-yloxy)phenyl]-3- methoxyacrylate is also increased.

### Detailed description of the invention

In order to further understanding the present invention, preferred embodiments of the present invention were described in combination with the following examples, however, it should be appreciated that these descriptions were only used to further illustrate the characteristics and advantages of the present invention, but not construed to limit the claims of the present invention.

A method for preparing (E)-methyl 2-[2-(6-chloropyrimidin-4-yloxy) phenyl]-3-methoxyacrylate is disclosed in the examples of the present invention, which comprises:
a. calcining, grinding and heating a basic catalyst to activate it, obtaining an activated catalyst;
b. performing a substitution reaction between methyl 3,3-dimethoxy-2-(2- hydroxyphenyl)propionate and 4,6-dichloropyrimidine in the presence of said activated catalyst, to obtain methyl 2-[2-(6-chloropyrimidin-4-yloxy)phenyl]-3,3- dimethoxypropionate; and
c. dealcoholizing the obtained methyl 2-[2-(6-chloropyrimidin-4-yloxy) phenyl]-3,3-dimethoxypropionate, to obtain (E)-methyl 2-[2-(6-chloropyrimidin -4-yloxy) phenyl]-3-methoxyacrylate.

Step a is a process for activating the catalyst, the activating process involves first calcining, grinding and heating the basic catalyst to activate it. Since the substitution reaction of the step b requires to be performed under anhydrous condition, the basic catalyst is firstly calcinized to remove the crystal water within the catalyst, so as to avoid the influence of water in the catalyst on the reaction, and to avoid the participation of water in the subsequent reactions; while the catalyst also forms a loose porous structure during the calcining, which also contributes to increase the contact area between the catalyst and the starting materials. The temperature of the calcining is preferably 300°C~450°C, excessively high temperature can easily cause decomposition of the catalyst. The duration of the calcining is preferably 2.5h to 4h, in order to achieve better dehydrating effects. Because the reaction of step b is a solid-liquid two-phase system, the contact area between the starting materials and the reactants can be increased after grinding the catalyst. Preferably, the basic catalyst after calcining is grinded to 50 mesh to 120 mesh; the grinded basic catalyst is heated to increase the activation energy of the basic catalyst, which improves the catalytic efficiency, preferably, the activation of the catalyst is performed under a condition of 70°C~130°C and 20mmHg-50mmHg.

The equation of step b is as follows:

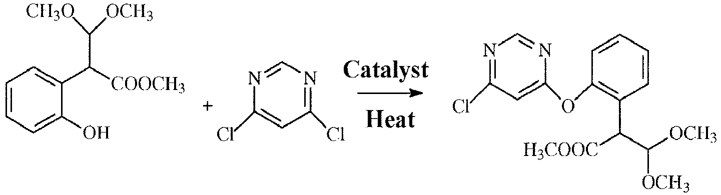

After activating the basic catalyst according to the above method, the catalytic efficiency of the catalyst is improved due to the increase of the activation energy, and in turn the conversion rates of the starting materials within the same reaction time were improved, and the product yield of step b is thus increased.

The molar ratio of methyl 3,3-dimethoxy-2-(2-hydroxyphenyl)propionate, 4,6-dichloropyrimidine to the activated catalyst is preferably 1:1.2~2.2:1.2~2.2, and the temperature of the substitution reaction is preferably 50°C~80°C.

After the substitution reaction, the product is purified by methods well known to the skilled in the art to obtain methyl 2-[2-(6-chloropyrimidin-4-yloxy) phenyl]-3,3-dimethoxypropionate.

The methyl 2-[2-(6-chloropyrimidin-4-yloxy)phenyl]-3,3-dimethoxypropionate prepared in step b is subjected to a dealcoholizing reaction, the equation of step c is as follows:

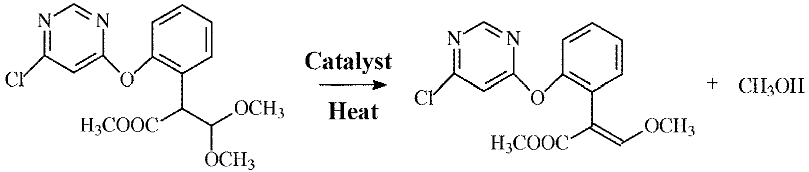

The temperature of this step is preferably controlled at 150°C~180°C, the pressure thereof is 15mmHg~25mmHg, and the catalyst employed is preferably selected from the group consisting of K₂CO₃, Na₂CO₃, NaHCO₃, KHSO₄ and KHCO₃. The mixture obtained after the reaction is dissolved with organic solvent, filtered to remove impurities. The filtrate after filtration is washed by water, dried and organic solvents were removed, to obtain (E)-methyl 2-[2-(6-chloropyrimidin-4-yloxy) phenyl]-3-methoxyacrylate.

The purity of product obtained in step c is still low, and the (E)-methyl 2-[2-(6-chloropyrimidin-4-yloxy) phenyl]-3-methoxyacrylate obtained in step c is hereinafter referred to as crude product, preferably, the above crude product is subjected to further purifying treatment according to the following method to improve the product purity:
d1. The crude product is subjected to decolorizing treatment;
d2. The decolorized (E)-methyl 2-[2-(6-chloropyrimidin-4-yloxy)phenyl]-3-methoxyacrylate is dissolved in a mixed solution of an ester compound and an alkane compound, and cooled for crystallization, said ester compound is isopropyl acetate or ethyl acetate, said alkane compound is cyclohexane or n-hexane, the weight ratio of the ester compound to the alkane compound in the mixed solution is 1.4~2.8:1, and the weight ratio of the decolorized (E)-methyl 2-[2-(6-chloropyrimidin-4-yloxy)phenyl]-3-methoxyacrylate to the mixed solution is 1:0.5~1.2.

In the above purifying method, the crude product is first subjected to decolorizing treatment, the purpose of which is to improve the effect of the subsequent crystallization, and to avoid the precipitation of the impurities along with the product during the crystallization. Preferably, the crude product is dissolved in isopropyl acetate or ethyl acetate, and activated charcoal is added to perform the decolorizing treatment, to remove the colored matter attached to the surface of the product by using the adsorbability of activated charcoal. The amount of activated charcoal added is preferably 5wt%-25wt% of the crude product by weight in order to secure sufficient dissolution of the crude product. After the addition of the activated charcoal, the crude product is heated under reflux for 0.5h-1.5h, then the activated charcoal is filtered out. The impurities adsorbed by the activated charcoal were also removed as the activated charcoal being filtered out.

Suitable solvents for crystallization should be selected during the crystallization process. (E)-methyl 2-[2-(6-chloropyrimidin-4-yloxy) phenyl]-3-methoxyacrylate has higher solubility in hot solvents, while the solubility thereof decreases greatly in cold solvents. Isopropyl acetate and ethyl acetate still have high solvency for (E)-methyl 2-[2-(6-chloropyrimidin-4-yloxy)phenyl]-3-methoxyacrylate even in low temperature, cyclohexane or n-hexane were thus added to the solvent(s) for crystallization in the present invention, to achieve that the solubilities of (E)-methyl 2-[2-(6-chloropyrimidin-4-yloxy)phenyl]-3-methoxyacrylate at different temperatures differ greatly while the solubilities of the impurities at different temperatures differ lesser in the solvent(s) for crystallization so as to avoid the precipitation of impurities together with the product during the crystallization, by adjusting the proportion of the above ester compound (isopropyl acetate or ethyl acetate) and alkane compound (cyclohexane or n-hexane) in the solvent(s) for crystallization. The weight ratio of the ester compound to the alkane compound in the solvent(s) for crystallization is 1.4~2.8:1, preferably 1.8~2.5:1, and the weight ratio between the decolorized (E)-methyl 2-[2-(6-chloropyrimidin-4-yloxy) phenyl]-3-methoxyacrylate and the solvent(s) for crystallization is 1:0.5~1.2, preferably 1:0.8~0.9. Collecting the crystals after cooling for crystallization to obtain (E)-methyl 2-[2-(6-chloropyrimidin-4-yloxy)phenyl]-3-methoxyacrylate of higher purity.

In order to further understand the present invention, the method for preparing (E)-methyl 2-[2-(6-chloropyrimidin-4-yloxy)phenyl]-3-methoxyacrylate provided in the present invention will be described in combination with the following examples.

### Example 1

1. K₂CO₃ was placed in a muffle, calcinated for 3.5h at 360°C and grinded to 120 mesh. The grinded K₂CO₃ was maintained at 30mmHg and 95°C for 3.5h to obtain activated catalyst.
2. DMF, 1 mol of methyl 3,3-dimethoxy-2-(2-hydroxyphenyl)propionate, 2 mol of 4,6-dichloropyrimidine and 2 mol of activated catalyst prepared in step 1 were added into the reaction vessel, and the reaction vessel was heated to 60°C to react for 8h before completing the reaction. The solution within the reaction vessel was filtered, and the filtrate was distilled under reduced pressure to remove DMF, then the mixture after distillation was dissolved in ethyl acetate and filtered, the filtrate was washed by water, dried, filtered, distilled under normal pressure, distilled under reduced pressure, to obtain methyl 2-[2-(6-chloropyrimidin-4-yloxy)phenyl]-3,3-dimethoxypropionate.
3. NaHSO₄ was added into the methyl 2-[2-(6-chloropyrimidin-4-yloxy) phenyl]-3,3-dimethoxypropionate obtained in step 1, and the reaction was performed for 2 hours under 160°C and 20mmHg. The mixture obtained in the reaction was dissolved in dichloromethane and filtered, the filtrate was washed by water, dried, filtered, distilled under normal pressure, distilled under reduced pressure to remove dichloromethane, to obtain the crude product of (E)-methyl 2-[2-(6-chloropyrimidin-4-yloxy)phenyl]-3-methoxyacrylate.
4. The crude product of (E)-methyl 2-[2-(6-chloropyrimidin-4-yloxy) phenyl]-3-methoxyacrylate obtained in step 2 was dissolved in isopropyl acetate, and activated charcoal was then added. The weight ratio of isopropyl acetate to the crude product was 3.5:1, the weight ratio of the activated charcoal to the crude product was 0.1:1. The mixed solution was heated under reflux for 1h and filtered to remove the activated charcoal, the filtrate was distilled under reduced pressure to a weight 1.5 times of that of the crude product. Cyclohexane was added and the weight ratio of cyclohexane to the crude product was 0.25:1. The solution was cooled for crystallization and crystals were collected. The purity of (E)-methyl 2-[2-(6-chloropyrimidin-4-yloxy)phenyl]-3-methoxyacrylate in the crystals was assayed to be 91.7%, and the yield was 88.5%.

### Example 2

1. Na₂CO₃ was placed in a muffle, calcinated for 3.5h at 320°C and grinded to 100 mesh, the grinded Na₂CO₃ was maintained at 30mmHg and 80°C for 3.5h to obtain activated catalyst.
2. DMF (N,N-dimethylformamide), 2 mol of methyl 3,3-dimethoxy-2-(2-hydroxyphenyl) propionate, 3.2 mol of 4,6-dichloropyrimidine and 3.2 mol of activated catalyst prepwered in step 1 were added into the reaction vessel, and the reaction vessel was heated to 60°C to react for 8h before completing the reaction. The solution within the reaction vessel was filtered, and the filtrate was distilled under reduced pressure to remove DMF, then the mixture after distillation was dissolved in ethyl acetate and filtered, the filtrate was washed by water, dried, filtered, distilled under normal pressure, distilled under reduced pressure, to obtain methyl 2-[2-(6-chloropyrimidin-4-yloxy)phenyl]-3,3-dimethoxypropionate.
3. KHSO₄ was added into the methyl 2-[2-(6-chloropyrimidin-4-yloxy) phenyl]-3,3-dimethoxypropionate obtained in step 2, and the reaction was performed for 2 hours under 160°C and 20mmHg. The mixture obtained in the reaction was dissolved in dichloromethane and filtered, the filtrate was washed by water, dried, filtered, distilled under normal pressure, distilled under reduced pressure to remove dichloromethane, so as to obtain the crude product of (E)-methyl 2-[2-(6-chloropyrimidin-4-yloxy)phenyl]-3-methoxyacrylate, the purity of (E)-methyl 2-[2-(6-chloropyrimidin-4-yloxy) phenyl]-3-methoxyacrylate in the product was assayed to be 80.6%, and the yield was 89.1 %.

### Example 3

1. KHCO₃ was placed in a muffle, calcinated for 2.5h at 450°C and grinded to 100 mesh, the grinded KHCO₃ was maintained at 50mmHg and 120°C for 3.5h to obtain activated catalyst.
2. DMF, 1 mol of methyl 3,3-dimethoxy-2-(2-hydroxyphenyl)propionate, 1.8 mol of 4,6-dichloropyrimidine and 1.8 mol of activated catalyst prepared in step 1 were added into the reaction vessel, and the reaction vessel was heated to 50°C to react for 8h before completing the reaction. The solution within the reaction vessel was filtered, and the filtrate was distilled under reduced pressure to remove DMF, then the mixture after distillation was dissolved in ethyl acetate and filtered, the filtrate was washed by water, dried, filtered, distilled under normal pressure, distilled under reduced pressure, to obtain methyl 2-[2-(6-chloropyrimidin-4-yloxy)phenyl]-3,3-dimethoxypropionate.
3. KHCO₃ was added into the methyl 2-[2-(6-chloropyrimidin-4-yloxy) phenyl]-3,3-dimethoxypropionate obtained in step 2, and the reaction was performed for 2 hours under 150°C and 18mmHg. The mixture obtained in the reaction was dissolved in dichloromethane and filtered, the filtrate was washed by water, dried, filtered, distilled under normal pressure, distilled under reduced pressure to remove dichloromethane, so as to obtain the crude product of (E)-methyl 2-[2-(6-chloropyrimidin-4-yloxy)phenyl]-3-methoxyacrylate.
4. The crude product of (E)-methyl 2-[2-(6-chloropyrimidin-4-yloxy) phenyl]-3-methoxyacrylate obtained in step 3 was dissolved in isopropyl acetate, and activated charcoal was then added. The weight ratio of isopropyl acetate to the crude product was 3.6:1, and the weight ratio of the activated charcoal to the crude product was 0.2:1. The mixed solution was heated under reflux for 1 h and filtered to remove the activated charcoal, the filtrate was distilled under reduced pressure to a weight 1.5 times of that of the crude product. Cyclohexane was added and the weight ratio of cyclohexane to the crude product was 0.2:1. The solution was cooled for crystallization and crystals were collected. The purity of (E)-methyl 2-[2-(6-chloropyrimidin-4-yloxy)phenyl]-3-methoxyacrylate in the crystals was assayed to be 92.5%, and the yield was 87.8%.

### Example 4

1. K₂CO₃ was placed in a muffle, calcinated for 3h at 350°C and grinded to 100 mesh, the grinded K₂CO₃ was maintained at 30mmHg and 100°C for 3.5h to obtain activated catalyst.
2. DMF, 1 mol of methyl 3,3-dimethoxy-2-(2-hydroxyphenyl)propionate, 1.6 mol of 4,6-dichloropyrimidine and 1.6 mol of activated catalyst prepared in step 1 were added into the reaction vessel, and the reaction vessel was heated to 70°C to react for 8h before completing the reaction. The solution within the reaction vessel was filtered, and the filtrate was distilled under reduced pressure to remove DMF, then the mixture after distillation was dissolved in ethyl acetate and filtered, the filtrate was washed by water, dried, filtered, distilled under normal pressure, distilled under reduced pressure, to obtain methyl 2-[2-(6-chloropyrimidin-4-yloxy)phenyl]-3,3-dimethoxypropionate.
3. KHSO₄ was added into the methyl 2-[2-(6-chloropyrimidin-4-yloxy) phenyl]-3,3-dimethoxypropionate obtained in step 2, and the reaction was performed for 2 hours under 170°C and 18mmHg. The mixture obtained in the reaction was dissolved in dichloromethane and filtered, the filtrate was washed by water, dried, filtered, distilled under normal pressure, distilled under reduced pressure to remove dichloromethane, so as to obtain the crude product of (E)-methyl 2-[2-(6-chloropyrimidin-4-yloxy)phenyl]-3-methoxyacrylate.
4. The crude product of (E)-methyl 2-[2-(6-chloropyrimidin-4-yloxy) phenyl]-3-methoxyacrylate obtained in step 3 was dissolved in isopropyl acetate, and activated charcoal was then added. The weight ratio of isopropyl acetate to the crude product was 3.5:1, and the weight ratio of the activated charcoal to the crude product was 0.1:1. The mixed solution was heated under reflux for 1h and filtered to remove the activated charcoal, the filtrate was distilled under reduced pressure to a weight 1.6 times of that of the crude product. Cyclohexane was added and the weight ratio between cyclohexane and the crude product was 0.3:1. The solution was cooled for crystallization and crystals were collected, the purity of (E)-methyl 2-[2-(6-chloropyrimidin-4-yloxy) phenyl]-3-methoxyacrylate in the crystals was assayed to be 91.9%, and the yield was 87.6%.

### Comparative example 1

1. DMF, 1 mol of methyl 3,3-dimethoxy-2-(2-hydroxyphenyl)propionate, 2.2 mol of 4,6-dichloropyrimidine and 2 mol of KHSO₄ were added into the reaction vessel, and the reaction vessel was heated to 60°C to react for 8h before completing the reaction. The solution within the reaction vessel was filtered, and the filtrate was distilled under reduced pressure to remove DMF, then the mixture after distillation was dissolved in ethyl acetate and filtered, the filtrate was washed by water, dried, filtered, distilled under normal pressure, distilled under reduced pressure, to obtain methyl 2-[2-(6-chloropyrimidin-4-yloxy)phenyl]-3,3-dimethoxypropionate.
2. NaHSO₄ was added into the methyl 2-[2-(6-chloropyrimidin-4-yloxy) phenyl]-3,3-dimethoxypropionate obtained in step 1, and the reaction was performed for 2 hours under 160°C and 20mmHg. The mixture obtained in the reaction was dissolved in dichloromethane and filtered, the filtrate was washed by water, dried, filtered, distilled under normal pressure, distilled under reduced pressure to remove dichloromethane, so as to obtain (E)-methyl 2-[2-(6-chloropyrimidin-4-yloxy)phenyl]-3-methoxyacrylate, the purity of (E)-methyl 2-[2-(6-chloropyrimidin-4-yloxy)phenyl]-3-methoxyacrylate in the product was assayed to be 78.5%, and the yield was 80.1%.

As was known from the above results, the yield of the product within the same reaction time can reach 88.1 % by preparing (E)-methyl 2-[2-(6-chloropyrimidin-4-yloxy) phenyl]-3-methoxyacrylate using the method of the present invention. The purity of the product can reach over 90%, if the crude product is subjected to further decolorizing and crystallizing treatments.

The illustrations of the above examples are merely used to help understand the method and the core idea of the present invention. It should be noted that for a skilled in the art a number of improvements and modifications can further be made to the present invention without departing from the principle of the present invention, and these improvements and modifications also fall within the scope claimed by the claims of present invention.

The above illustrations of the disclosed examples will enable a skilled in the art to realize or utilize the present invention. A variety of modifications of these examples will be apparent to a skilled in the art, and the general principle defined herein can be realized in other examples without departing the sprit or scope of the present invention. Therefore, the present invention will not be limited to the examples illustrated herein, but to be in line with the broadest scope which is consistent with the principle and the novel characteristics disclosed herein.

## Claims

1. A method for preparing (E)-methyl 2-[2-(6-chloropyrimidin-4-yloxy)phenyl]-3-methoxyacrylate, **characterized in that** the method comprises:
a. calcining, grinding and heating a basic catalyst to activate it, obtaining an activated catalyst;
b. performing a substitution reaction between methyl 3,3-dimethoxy-2-(2-hydroxyphenyl)propionate and 4,6-dichloropyrimidine in the presence of said activated catalyst, to obtain methyl 2-[2-(6-chloropyrimidin-4-yloxy)phenyl]-3,3-dimethoxypropionate; and
c. dealcoholizing the obtained methyl 2-[2-(6-chloropyrimidin-4-yloxy)phenyl]-3,3-dimethoxypropionate, to obtain (E)-methyl 2-[2-(6-chloropyrimidin -4-yloxy) phenyl]-3-methoxyacrylate; **characterized in that** the temperature of the calcining is 300°C-450°C and that the duration of the calcining is 2.5h~4h, wherein the activated catalyst is selected from the group consisting of K₂CO₃, Na₂CO₃, NaHCO₃, KHSO₄ and KHCO₃.

2. The method according to claim 1, **characterized in that** the temperature of the heating activation is 70°C ~130°C, and the pressure thereof is 20mmHg~50mmHg.

3. The method according to claim 1, **characterized in that** the particle size of the activated catalyst is 50 mesh~124 mesh.

4. The method according to claim 1, **characterized in that** the molar ratio of methyl 3,3-dimethoxy-2-(2-hydroxyphenyl)propionate, 4,6-dichloropyrimidine and the activated catalyst is 1:1.2~2.2:1.2~2.2.

5. The method according to claim 1, **characterized in that** the method further comprises a step of purifying (E)-methyl 2-[2-(6-chloropyrimidin-4-yloxy) phenyl]-3-methoxyacrylate, said purifying is consisted of:
performing a decolorizing treatment to (E)-methyl 2-[2-(6-chloropyrimidin-4-yloxy) phenyl]-3-methoxyacrylate;
dissolving the decolorized (E)-methyl 2-[2-(6-chloropyrimidin-4-yloxy) phenyl]-3-methoxyacrylate using a mixed solution of an ester compound and an alkane compound, and cooling the same for crystallization, wherein the ester compound is isopropyl acetate or ethyl acetate, the alkane compound is cyclohexane or n-hexane, the weight ratio of the ester compound to the alkane compound in the mixed solution is 1.4~2.8:1, and the weight ratio of the decolorized (E)-methyl 2-[2-(6-chloropyrimidin-4-yloxy)phenyl]-3-methoxyacrylate to the mixed solution is 1:0.5~1.2.

6. The method according to claim 5, **characterized in that** the decolorizing treatment consists of the following steps: dissolving (E)-methyl 2-[2-(6-chloropyrimidin-4-yloxy)phenyl]-3-methoxyacrylate using isopropyl acetate or ethyl acetate, and then adding activated charcoal.

7. The method according to claim 6, **characterized in that** the weight ratio of (E)-methyl 2-[2-(6-chloropyrimidin-4-yloxy)phenyl]-3-methoxyacrylate to the activated charcoal is 1:0.05~0.25.

## Patentansprüche

1. Verfahren zur Darstellung von (E)-Methyl 2-[2-(6-chlorpyrimidin-4-yloxy)phenyl]-3-methoxyacrylat, **dadurch gekennzeichnet, dass** das Verfahren aufweist:
a. Calcinieren, Mahlen und Erhitzen eines basischen Katalysators zu dessen Aktivierung, wobei ein aktivierter Katalysator erhalten wird;
b. Durchführen einer Substitutionsreaktion zwischen Methyl 3,3-dimethoxy-2-(2-hydroxyphenyl)propionat und 4,6-Dichlorpyrimidin in der Anwesenheit des aktivierten Katalysators, um Methyl 2-[2-(6-chlorpyrimidin-4-yloxy)phenyl]-3,3-dimethoxypropionat zu erhalten; und
c. Dealkoholisieren des erhaltenen Methyl 2-[2-(6-chlorpyrimidin-4-yloxy)phenyl]-3,3-dimethoxypropionats, um (E)-Methyl 2-[2-(6-chlorpyrimidin-4-yloxy)phenyl]-3-methoxyacrylat zu erhalten; **dadurch gekennzeichnet, dass**
die Temperatur des Calcinierens 300°C - 450°C beträgt und dass die Dauer des Calcinierens 2,5 h ~ 4 h beträgt, wobei der aktivierte Katalysator ausgewählt ist unter der Gruppe bestehend aus K₂CO₃, Na₂CO₃, NaHCO₃, KHSO₄ und KHCO₃.

2. Verfahren nach Anspruch 1, **dadurch gekennzeichnet, dass** die Temperatur der Hitzeaktivierung 70 °C ~ 130 °C beträgt, und dass der Druck davon 20 mmHg ~ 50 mmHg beträgt.

3. Verfahren nach Anspruch 1, **dadurch gekennzeichnet, dass** die Teilchengröße des aktivierten Katalysators 50 Mesh ~120 Mesh beträgt.

4. Verfahren nach Anspruch 1, **dadurch gekennzeichnet, dass** das Molverhältnis des Methyl 3,3-dimethoxy-2-(2-hydroxyphenyl)propionats, 4,6-Dichlorpyrimidins und des aktivierten Katalysators 1 : 1,2 ~ 2,2 :1,2 ~ 2,2 beträgt.

5. Verfahren nach Anspruch 1, **dadurch gekennzeichnet, dass** das Verfahren ferner einen Reinigungsschritt von (E)-Methyl 2-[2-(6-chlorpyrimidin-4-yloxy)phenyl]-3-methoxyacrylat aufweist, wobei das Reinigen aufweist:
(E)-Methyl 2-[2-(6-chlorpyrimidin-4-yloxy)phenyl]-3-methoxyacrylat einer Entfärbungsbehandlung unterziehen;
Lösen des entfärbten (E)-Methyl 2-[2-(6-chlorpyrimidin-4-yloxy)phenyl]-3-methoxyacrylats unter Verwendung einer Mischlösung einer Esterverbindung und einer Alkanverbindung, und Abkühlen derselben zur Kristallisation, wobei die Esterverbindung Isopropylacetat oder Ethylacetat ist, die Alkanverbindung Cyclohexan oder n-Hexan ist, das Gewichtsverhältnis der Esterverbindung zu der Alkanverbindung in der Mischlösung 1,4 ~ 2,8 : 1 beträgt, und das Gewichtsverhältnis des entfärbten (E)-Methyl 2-[2-(6-chlorpyrimidin-4-yloxy)phenyl]-3-methoxyacrylats zu der Mischlösung 1 : 0,5 ~ 1,2 beträgt.

6. Verfahren nach Anspruch 5, **dadurch gekennzeichnet, dass** die Entfärbungsbehandlung die nachstehend aufgeführten Schritte aufweist: Lösen von (E)-Methyl 2-[2-(6-chlorpyrimidin-4-yloxy)phenyl]-3-methoxyacrylat unter Verwendung von Isopropylacetat oder Ethylacetat, und anschließendes Hinzufügen von Aktivkohle.

7. Verfahren nach Anspruch 6, **dadurch gekennzeichnet, dass** das Gewichtsverhältnis von (E)-Methyl 2-[2-(6-chlorpyrimidin-4-yloxy)phenyl]-3-methoxyacrylat zu der Aktivkohle 1 : 0,05 ~ 0,25 beträgt.

## Revendications

1. Procédé de préparation du 2-[2-(6-chloropyrimidine-4-yloxy)phényle]-3-méthoxyacrylate de (E)-méthyle, **caractérisé en ce que** le procédé comprend :
a. calciner, broyer et chauffer un catalyseur basique afin de l'activer, obtenir un catalyseur activé ;
b. réaliser une réaction de substitution entre le 3,3-diméthoxy-2-(2-hydroxyphényle)propionate de méthyle et la 4,6-dichloropyrimidine en présence dudit catalyseur activé, afin d'obtenir le 2-[2-(6-chloropyrimidine-4-yloxy)phényle]-3,3-diméthoxypropionate de méthyle ; et
c. éliminer un groupement alcool du 2-[2-(6-chloropyrimidine-4-yloxy)phényle]-3,3-diméthoxypropionate de méthyle obtenu, afin d'obtenir le 2-[2-(6-chloropyrimidine-4-yloxy)phényle]-3-méthoxyacrylate de (E)-méthyle ; **caractérisé en ce que** la température de la calcination est de 300 °C à 450 °C et **en ce que** la durée de la calcination est de 2,5 h à 4 h, le catalyseur activé étant sélectionné à partir du groupe constitué de K₂CO₃, Na₂CO₃, NaHCO₃, KHSO₄ et KHCO₃.

2. Procédé selon la revendication 1, **caractérisé en ce que** la température de l'activation par chauffage est de 70 °C à 130 °C, et la pression lors de cette activation est comprise entre 20 mmHg et 50 mmHg.

3. Procédé selon la revendication 1, **caractérisé en ce que** la taille des particules du catalyseur activé est de 50 mesh à 120 mesh.

4. Procédé selon la revendication 1, **caractérisé en ce que** le rapport molaire du 3,3-diméthoxy-2-(2-hydroxyphényle)propionate de méthyle, de la 4,6-dichloropyrimidine et du catalyseur activé est de 1:1,2 à 2,2:1,2 à 2,2.

5. Procédé selon la revendication 1, **caractérisé en ce que** le procédé comprend en outre une étape de purification du 2-[2-(6-chloropyrimidine-4-yloxy)phényle]-3-méthoxyacrylate de (E)-méthyle, ladite purification comprenant :
réaliser un traitement de décoloration sur le 2-[2-(6-chloropyrimidine-4-yloxy)phényle]-3-méthoxyacrylate de (E)-méthyle ;
dissoudre du 2-[2-(6-chloropyrimidine-4-yloxy)phényle]-3-méthoxyacrylate de (E)-méthyle décoloré à l'aide d'une solution mixte constituée d'un composé ester et d'un composé alcane, et refroidir la solution ainsi obtenue pour une cristallisation, le composé ester étant l'acétate d'isopropyle ou l'acétate d'éthyle, le composé alcane est le cyclohexane ou le n-hexane, le rapport massique du composé ester sur le composé alcane dans la solution mixte est de 1,4-2,8:1, et le rapport massique du 2-[2-(6-chloropyrimidine-4-yloxy)phényle]-3-méthoxyacrylate de (E)-méthyle décoloré sur la solution mixte est de 1:0,05 à 1,2.

6. Procédé selon la revendication 5, **caractérisé en ce que** le traitement de décoloration comprend les étapes suivantes : dissoudre du 2-[2-(6-chloropyrimidine-4-yloxy)phényle]-3-méthoxyacrylate de (E)-méthyle à l'aide d'acétate d'isopropyle ou d'acétate d'éthyle, et ensuite ajouter le charbon actif activé.

7. Procédé selon la revendication 6, **caractérisé en ce que** le rapport massique du 2-[2-(6-chloropyrimidine-4-yloxy)phényle]-3-méthoxyacrylate de (E)-méthyle sur le charbon actif activé est de 1:0,05 à 0,25.
